# EUROPEAN PATENT SPECIFICATION

(11) **EP 0 727 990 B1**
(45) Date of publication and mention of the grant of the patent: **13.06.2001**
(21) Application number: 94931286.2
(22) Date of filing: 23.09.1994
(51) Int. Cl.: A61K 31/275, C07C 255/46

(54) **COMPOUNDS, COMPOSITIONS AND TREATMENT OF ALLERGIES AND INFLAMMATION**
VERBINDUNGEN, ZUBEREITUNGEN UND BEHANDLUNG VON ALLERGIEN UND ENTZÜNDUNGEN
COMPOSES, COMPOSITIONS ET TRAITEMENT D'ALLERGIES ET DE L'INFLAMMATION

(30) Priority: 01.10.1993 US 130215
(43) Date of publication of application: 28.08.1996
(73) Proprietor: SMITHKLINE BEECHAM CORPORATION, Philadelphia Pennsylvania 19101 (US)
(72) Inventor: CHRISTENSEN, Siegfried, Benjamin, IV, Philadelphia, PA 19103 (US); WEBB, Kevin, Scott, Phoenixville, PA 19460 (US)
(74) Representative: Rutter, Keith, Dr.
(86) International application number: US9410815
(87) International publication number: WO9509624

(56) References cited:
- WO-A-93/19748
- WO-A-93/19750
- WO-A-95/03794
- CHEM. PHARM. BULL., Vol. 24(7), issued 1976, TAKEDA et al., "Azabicycloalkanes as Analgetics. II 1 An Improved Synthesis of 1-Phenyl-6-Azabicyclo(3,2,1)-Octane Derivatives", pages 1514-1526.

## Description

The present invention relates to novel compounds, pharmaceutical compositions containing these compounds, and their use in treating allergic and inflammatory diseases and for inhibiting the production of Tumor Necrosis Factor (TNF).

### Background of the Invention

Bronchial asthma is a complex, multifactorial disease characterized by reversible narrowing of the airway and hyperreactivity of the respiratory tract to external stimuli.

Identification of novel therapeutic agents for asthma is made difficult by the fact that multiple mediators are responsible for the development of the disease. Thus, it seems unlikely that eliminating the effects of a single mediator will have a substantial effect on all three components of chronic asthma. An alternative to the "mediator approach" is to regulate the activity of the cells responsible for the pathophysiology of the disease.

One such way is by elevating levels of cAMP (adenosine cyclic 3',5'-monophosphate). Cyclic AMP has been shown to be a second messenger mediating the biologic responses to a wide range of hormones, neurotransmitters and drugs; [Krebs Endocrinology Proceedings of the 4th International Congress Excerpta Medica, 17-29, 1973]. When the appropriate agonist binds to specific cell surface receptors, adenylate cyclase is activated, which converts Mg⁺²-ATP to cAMP at an accelerated rate.

Cyclic AMP modulates the activity of most, if not all, of the cells that contribute to the pathophysiology of extrinsic (allergic) asthma. As such, an elevation of cAMP would produce beneficial effects including: 1) airway smooth muscle relaxation, 2) inhibition of mast cell mediator release, 3) suppression of neutrophil degranulation, 4) inhibition of basophil degranulation, and 5) inhibition of monocyte and macrophage activation. Hence, compounds that activate adenylate cyclase or inhibit phosphodiesterase should be effective in suppressing the inappropriate activation of airway smooth muscle and a wide variety of inflammatory cells. The principal cellular mechanism for the inactivation of cAMP is hydrolysis of the 3'-phosphodiester bond by one or more of a family of isozymes referred to as cyclic nucleotide phosphodiesterases (PDEs).

It has now been shown that a distinct cyclic nucleotide phosphodiesterase (PDE) isozyme, PDE IV, is responsible for cAMP breakdown in airway smooth muscle and inflammatory cells. [Torphy, "Phosphodiesterase Isozymes: Potential Targets for Novel Anti-asthmatic Agents" in New Drugs for Asthma, Barnes, ed. IBC Technical Services Ltd., 1989]. Research indicates that inhibition of this enzyme not only produces airway smooth muscle relaxation, but also suppresses degranulation of mast cells, basophils and neutrophils along with inhibiting the activation of monocytes and neutrophils. Moreover, the beneficial effects of PDE IV inhibitors are markedly potentiated when adenylate cyclase activity of target cells is elevated by appropriate hormones or autocoids, as would be the case *in vivo.* Thus PDE IV inhibitors would be effective in the asthmatic lung, where levels of prostaglandin E₂ and prostacyclin (activators of adenylate cyclase) are elevated. Such compounds would offer a unique approach toward the pharmacotherapy of bronchial asthma and possess significant therapeutic advantages over agents currently on the market.

The compounds of this invention also inhibit the production of Tumor Necrosis Factor (TNF), a serum glycoprotein. Excessive or unregulated TNF production has been implicated in mediating or exacerbating a number of diseases including rheumatoid arthritis, rheumatoid spondylitis, osteoarthritis, gouty arthritis and other arthritic conditions; sepsis, septic shock, endotoxic shock, gram negative sepsis, toxic shock syndrome, adult respiratory distress syndrome, cerebral malaria, chronic pulmonary inflammatory disease, silicosis, pulmonary sarcoidosis, bone resorption diseases, reperfusion injury, graft vs. host reaction, allograft rejections, fever and myalgias due to infection, such as influenza, cachexia secondary to infection or malignancy, cachexia secondary to human acquired immune deficiency syndrome (AIDS), AIDS, ARC (AIDS related complex), keloid formation, scar tissue formation, Crohn's disease, ulcerative colitis, or pyresis, in addition to a number of autoimmune diseases, such as multiple sclerosis, autoimmune diabetes and systemic lupus erythematosis.

AIDS results from the infection of T lymphocytes with Human Immunodeficiency Virus (HIV). At least three types or strains of HIV have been identified, i.e., HIV-1, HIV-2 and HIV-3. As a consequence of HIV infection, T-cell-mediated immunity is impaired and infected individuals manifest severe opportunistic infections and/or unusual neoplasms. HIV entry into the T lymphocyte requires T lymphocyte activation. Viruses such as HIV-1 or HIV-2 infect T lymphocytes after T cell activation and such virus protein expression and/or replication is mediated or maintained by such T cell activation. Once an activated T lymphocyte is infected with HIV, the T lymphocyte must continue to be maintained in an activated state to permit HIV gene expression and/or HIV replication.

Cytokines, specifically TNF, are implicated in activated T-cell-mediated HIV protein expression and/or virus replication by playing a role in maintaining T lymphocyte activation. Therefore, interference with cytokine activity such as by inhibition of cytokine production, notably TNF, in an HIV-infected individual aids in limiting the maintenance of T cell activation, thereby reducing the progression of HIV infectivity to previously uninfected cells which results in a slowing or elimination of the progression of immune dysfunction caused by HIV infection. Monocytes, macrophages, and related cells, such as kupffer and glial cells, have also been implicated in maintenance of the HIV infection. These cells, like T cells, are targets for viral replication and the level of viral replication is dependent upon the activation state of the cells. [See Rosenberg *et al*., The Immunopathogenesis of HIV Infection, Advances in Immunology, Vol. 57, 1989]. Monokines, such as TNF, have been shown to activate HIV replication in monocytes and/or macrophages [See Poli *et al*., Proc. Natl. Acad. Sci., 87; 782-784, 1990], therefore, inhibition of monokine production or activity aids in limiting HIV progression as stated above for T cells.

TNF has also been implicated in various roles with other viral infections, such as the cytomegalovirus (CMV), influenza virus, adenovirus, and the herpes virus for similar reasons as those noted.

TNF is also associated with yeast and fungal infections. Specifically *Candida albicans* has been shown to induce TNF production *in vitro* in human monocytes and natural killer cells. [See Riipi *et al*., Infection and Immunity, 58(9):2750-54, 1990, and Jafari *et al*., Journal of Infectious Diseases, 164:389-95, 1991. See also Wasan *et al*., Antimicrobial Agents and Chemotherapy, 35,(10):2046-48, 1991; and Luke *et al*., Journal of Infectious Diseases, 162:211-214,1990].

The ability to control the adverse effects of TNF is furthered by the use of the compounds which inhibit TNF in mammals who are in need of such use. Compounds useful for the treatment of disease states mediated or exacerbated by TNF production are known from WO 95/03794, WO 93/19748 and WO 93/19750. There remains a need for further compounds which are useful in treating TNF-mediated disease states which are exacerbated or caused by the excessive and/or unregulated production of TNF.

### Summary of the Invention

This invention relates to the novel compounts of Formula (I) as shown below, useful in the mediation or inhibition of the enzymatic activity (or catalytic activity) of phosphodiesterase IV (PDE IV). These compounds also have Tumor Necrosis Factor (TNF) inhibitory activity.

This invention also relates to the pharmaceutical compositions comprising a compound of Formula (I) and a pharmaceutically acceptable carrier or diluent.

The invention also relates to a method of mediation or inhibition of the enzymatic activity (or catalytic activity) of PDE IV in mammals, including humans, which comprises administering to a mammal in need thereof an effective amount of a compound of Formula (I) as shown below.

The invention further provides a method for the treatment of allergic and inflammatory disease which comprises administering to a mammal, including humans, in need thereof, an effective amount of a compound of Formula (I).

The invention also provides a method for the treatment of asthma which comprises administering to a mammal, including humans, in need thereof, an effective amount of a compound of Formula (I).

This invention also relates to a method of inhibiting TNF production in a mammal, including humans, which method comprises administering to a mammal in need of such treatment, an effective TNF inhibiting amount of a compound of Formula (I). This method may be used for the prophylactic treatment or prevention of certain TNF mediated disease states amenable thereto.

This invention also relates to a method of treating a human afflicted with a human immunodeficiency virus (HIV), which comprises administering to such human an effective TNF inhibiting amount of a compound of Formula (I).

Compounds of Formula (I) are also useful in the treatment of additional viral infections, where such viruses are sensitive to upregulation by TNF or will elicit TNF production *in vivo*.

In addition, compounds of Formula (I) are also useful in treating yeast and fungal infections, where such yeast and fungi are sensitive to upregulation by TNF or will elicit TNF production *in vivo*.

Novel compounds of this invention are represented by Formula (I): 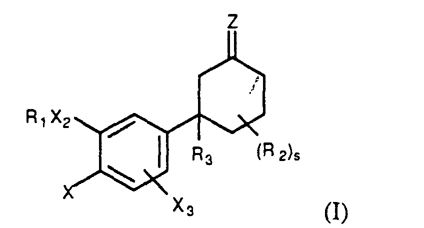 wherein:
R₁ is -(CR₄R₅)ₙC(O)O(CR₄R₅)ₘR₆, -(CR₄R₅)ₙC(O)NR₄(CR₄R₅)ₘR₆,-(CR₄R₅)ₙO(CR₄R₅)ₘR₆, or -(CR₄R₅)ᵣR₆ wherein the alkyl moieties may be optionally substituted with one or more halogens;
m is 0 to 2;
n is 1 to 4;
r is 0 to 6;
R₄ and R₅ are independently selected hydrogen or C₁₋₂ alkyl;
R₆ is hydrogen, methyl, hydroxyl, aryl, halo substituted aryl, aryloxyC₁₋₃ alkyl, halo substituted aryloxyC₁₋₃ alkyl, indanyl, indenyl, C₇₋₁₁ polycycloalkyl, tetrahydrofuranyl, furanyl, tetrahydropyranyl, pyranyl, tetrahydrothienyl, thienyl, tetrahydrothiopyranyl, thiopyranyl, C₃₋₆ cycloalkyl, or a C₄₋₆ cycloalkyl containing one or two unsaturated bonds, wherein the cycloalkyl and heterocyclic moieties may be optionally substituted by 1 to 3 methyl groups or one ethyl group;
provided that:
   a) when R₆ is hydroxyl, then m is 2; or
   b) when R₆ is hydroxyl, then r is 2 to 6; or
   c) when R₆ is 2-tetrahydropyranyl, 2-tetrahydrothiopyranyl, 2-tetrahydrofuranyl, or 2-tetrahydrothienyl, then m is 1 or 2; or
   d) when R₆ is 2-tetrahydropyranyl, 2-tetrahydrothiopyranyl, 2-tetrahydrofuranyl, or 2-tetrahydrothienyl, then r is 1 to 6;
   e) when n is 1 and m is 0, then R₆ is other than H in -(CR₄R₅)ₙO(CR₄R₅)ₘR₆;
X is YR₂, halogen, nitro, NR₄R₅, or formyl amine;
Y is O or S(O)_{m'};
m' is 0, 1, or 2;
X₂ is O or NR₈;
X₃ is hydrogen or X;
R₂ is independently selected from -CH₃ or -CH₂CH₃ optionally substituted by 1 or more halogens;
s is 0 to 4;
R₃ is C₁₋₄ alkyl, fluoro-substituted C₁₋₄ alkyl, CH₂NHC(O)C(O)NH₂,-CH=CR_{8'}R_{8'}, cyclopropyl optionally substituted by R_{8'}, CN, CH₂OR₈, CH₂NR₈R₁₀, C(Z')H, C(O)OR₈, C(O)NR₈R₁₀, or C≡CR_{8'};
Z' is O, NR₉, NOR₈, NCN, C(-CN)₂, CR₈CN, CR₈NO₂, CR₈C(O)OR₈, CR₈C(O)NR₈R₈, C(-CN)NO₂, C(-CN)C(O)OR₉, or C(-CN)C(O)NR₈R₈ ;
Z is O, NR₇, NCR₄R₅C₂₋₆ alkenyl, NOR₁₄, NOR₁₅, NOCR₄R₅C₂₋₆ alkenyl, NNR₄R₁₄, NNR₄R₁₅, NCN, NNR₈C(O)NR₈R₁₄, NNR₈C(S)NR₈R₁₄, C(-CN)₂, CR₁₄CN, CR₁₄C(O)OR₈, CR₁₄C(O)NR₈R₁₄, C(-CN)NO₂, C(-CN)C(O)OR₉, C(-CN)OC(O)R₉, C(-CN)OR₉, C(-CN)C(O)NR₈R₁₄, or =Z is 2-(1,3-dithiane), 2-(1,3-dithiolane), dimethylthio ketal, diethylthio ketal, 2-(1,3-dioxolane), 2(1,3-dioxane), 2-(1,3-oxathiolane), dimethyl ketal or diethyl ketal;
R₇ is -(CR₄R₅)_{q}R₁₂ or C₁₋₆ alkyl wherein the R₁₂ or C₁₋₆ alkyl group is optionally substituted one or more times by C₁₋₂ alkyl optionally substituted by one to three fluorines, -F, -Br, -Cl, -NO₂, -Si(R₄)₃, -NR₁₀R₁₁, -C(O)R₈, -CO₂R₈, -OR₈, -CN, -C(O)NR₁₀R₁₁, -OC(O)NR₁₀R₁₁, -OC(O)R₈, -NR₁₀C(O)NR₁₀R₁₁, -NR₁₀C(O)R₁₁, -NR₁₀C(O)OR₉, -NR₁₀C(O)R₁₃, -C(NR₁₀)NR₁₀R₁₁, -C(NCN)NR₁₀R₁₁,-C(NCN)SR₉, -NR₁₀C(NCN)SR₉, -NR₁₀C(NCN)NR₁₀R₁₁, -NR₁₀S(O)₂R₉, -S(O)_{m'}R₉, -NR₁₀C(O)C(O)NR₁₀R₁₁, -NR₁₀C(O)C(O)R₁₀, thiazolyl, imidazolyl, oxazolyl, pyrazolyl, triazolyl, or tetrazolyl;
q is 0, 1, or 2;
R₁₂ is C₃₋₇ cycloalkyl, (2-, 3- or 4-pyridyl), pyrimidyl, pyrazolyl, (1- or 2-imidazolyl), thiazolyl, triazolyl, pyrrolyl, piperazinyl, piperidinyl, morpholinyl, furanyl, (2- or 3-thienyl), (4- or 5-thiazolyl), quinolinyl, naphthyl, or phenyl;
R₈ is independently selected from hydrogen or R₉;
R_{8'} is R₈ or fluorine;
R₉ is C₁₋₄ alkyl optionally substituted by one to three fluorines;
R₁₀ is OR₈ or R₁₁;
R₁₁ is hydrogen, or C₁₋₄ alkyl optionally substituted by one to three fluorines; or when R₁₀ and R₁₁ are as NR₁₀R₁₁ they may together with the nitrogen form a 5 to 7 membered ring optionally containing at least one additional heteroatom selected from O, N, or S;
R₁₃ is oxazolidinyl, oxazolyl, thiazolyl, pyrazolyl, triazolyl, tetrazolyl, imidazolyl, imidazolidinyl, thiazolidinyl, isoxazolyl, oxadiazolyl, or thiadiazolyl, and each of these heterocyclic rings is connected through a carbon atom and each may be unsubstituted or substituted by one or two C₁₋₂ alkyl groups;
R₁₄ is hydrogen or R₇; or when R₈ and R₁₄ are as NR₈R₁₄ they may together with the nitrogen form a 5 to 7 membered ring optionally containing one or more additional heteroatoms selected from O, N, or S;
R₁₅ is C(O)R₁₄, C(O)NR₄R₁₄, S(O)₂R₇, or S(O)₂NR₄R₁₄; provided that:
   (f) when Z is O, X₂ is oxygen, X₃ is hydrogen, s is 0, and X is YR₂, then R₃ is other than hydrogen;
   (g) when R₁₂ is N-pyrazolyl, N-imidazolyl, N-triazolyl, N-pyrrolyl, N-piperazinyl, N-piperidinyl, or N-morpholinyl, then q is not 1; or
   (h) when Z is O or =Z is 2-(1,3-dioxolane) and R₃ is CH₃, CH₂OH or CH₂OC₁₋₄ alkyl, then R₁X₂ is not C₁-C₃ alkoxy and X is not halogen, methoxy, ethoxy, methylthio, Or ethylthio; not being 3-cyano-3-(3,4-dimethoxyphenyl) cyclohexan-1-one,
   or pharmaceutically acceptable salts thereof.

### Detailed Description of the Invention

This invention also relates to a method of mediating or inhibiting the enzymatic activity (or catalytic activity) of PDE IV in a mammal in need thereof and to inhibiting the production of TNF in a mammal in need thereof, which comprises administering to said mammal an effective amount of a compound of Formula (I).

Phosphodiesterase IV inhibitors are useful in the treatment of a variety of allergic and inflammatory diseases including: asthma, chronic bronchitis, atopic dermatitis, urticaria, allergic rhinitis, allergic conjunctivitis, vernal conjunctivitis, eosinophilic granuloma, psoriasis, rheumatoid arthritis, septic shock, ulcerative colitis, Crohn's disease, reperfusion injury of the myocardium and brain, chronic glomerulonephritis, endotoxic shock and adult respiratory distress syndrome. In addition, PDE IV inhibitors are useful in the treatment of diabetes insipidus and central nervous system disorders such as depression and multi-infarct dementia.

The viruses contemplated for treatment herein are those that produce TNF as a result of infection, or those which are sensitive to inhibition, such as by decreased replication, directly or indirectly, by the TNF inhibitors of Formula (I). Such viruses include, but are not limited to HIV-1, HIV-2 and HIV-3, cytomegalovirus (CMV), influenza, adenovirus and the Herpes group of viruses, such as, but not limited to, *Herpes zoster* and *Herpes simplex*.

This invention more specifically relates to a method of treating a mammal, afflicted with a human immunodeficiency virus (HIV), which comprises administering to such mammal an effective TNF inhibiting amount of a compound of Formula (I).

The compounds of this invention may also be used in association with the veterinary treatment of animals, other than in humans, in need of inhibition of TNF production. TNF mediated diseases for treatment, therapeutically or prophylactically, in animals include disease states such as those noted above, but in particular viral infections. Examples of such viruses include, but are not limited to feline immunodeficiency virus (FIV) or other retroviral infection such as equine infectious anemia virus, caprine arthritis virus, visna virus, maedi virus and other lentiviruses.

The compounds of this invention are also useful in treating yeast and fungal infections, where such yeast and fungi are sensitive to upregulation by TNF or will elicit TNF production *in vivo.* A preferred disease state for treatment is fungal meningitis. Additionally, the compounds of Formula (I) may be administered in conjunction with other drugs of choice for systemic yeast and fungal infections. Drugs of choice for fungal infections, include but are not limited to the class of compounds called the polymixins, such as Polymycin B, the class of compounds called the imidazoles, such as clotrimazole, econazole, miconazole, and ketoconazole; the class of compounds called the triazoles, such as fluconazole, and itranazole, and the class of compound called the Amphotericins, in particular Amphotericin B and liposomal Amphotericin B.

The compounds of Formula (I) may also be used for inhibiting and/or reducing the toxicity of an anti-fungal, anti-bacterial or anti-viral agent by administering an effective amount of a compound of Formula (I) to a mammal in need of such treatment. Preferably, a compound of Formula (I) is administered for inhibiting or reducing the toxicity of the Amphotericin class of compounds, in particular Amphotericin B.

Preferred compounds are as follows:

When R₁ for the compounds of Formula (I) is an alkyl substituted by 1 or more halogens, the halogens are preferably fluorine and chlorine, more preferably a C₁₋₄ alkyl substituted by 1 or more fluorines. The preferred halo-substituted alkyl chain length is one or two carbons, and most preferred are the moieties -CF₃, -CH₂F, -CHF₂, -CF₂CHF₂, -CH₂CF₃, and -CH₂CHF₂. Preferred R₁ substitutents for the compounds of Formula (I) are CH₂-cyclopropyl, CH₂-C₅₋₆ cycloalkyl, C₄₋₆ cycloalkyl, C₇₋₁₁ polycycloalkyl, (3- or 4-cyclopentenyl), phenyl, tetrahydrofuran-3-yl, benzyl or C₁₋₂ alkyl optionally substituted by 1 or more fluorines, -(CH₂)₁₋₃C(O)O(CH₂)₀₋₂CH₃, -(CH₂)₁₋₃O(CH₂)₀₋₂CH₃, and -(CH₂)₂₋₄OH.

When the R₁ term is (CR₄R₅), the R₄ and R₅ terms are independently hydrogen or alkyl. This allows for branching of the individual methylene units as (CR₄R₅)ₙ or (CR₄R₅)ₘ; each repeating methylene unit is independent of the other, e.g., (CR₄R₅)ₙ wherein n is 2 can be -CH₂CH(-CH₃)-, for instance. The individual hydrogen atoms of the repeating methylene unit or the branching hydrocarbon can optionally be substituted by fluorine independent of each other to yield, for instance, the preferred R₁ substitutions, as noted above.

When R₁ is a C₇₋₁₁ polycycloalkyl, examples are bicyclo[2.2.1]-heptyl, bicyclo[2.2.2]octyl, bicyclo[3.2.1]octyl, tricyclo[5.2.1.0^{2,6}]decyl, etc. additional examples of which are described in Saccamano *et al*., WO 87/06576, published 5 November 1987, whose disclosure is incorporated herein by reference in its entirety.

Preferred Z terms are O, NCN, NR₇, NOR₁₄, NOR₁₅, NNR₄R₁₄, NNR₄R₁₅,C(CN)₂, C(-CN)OC(O)R₉, C(-CN)OR₉, CR₁₄C(O)OR₈, CR₉C(O)NR₁₃R₁₄, 2-(1,3-dithiane), dimethylthio ketal, 2-(1,3-dioxolane), or dimethyl ketal. More preferred are O, NR₇, NOR₁₄, NOR₁₅, and 2-(1,3-dioxolane).

Preferred X groups for Formula (I) are those wherein X is YR₂ and Y is oxygen. The preferred X₂ group for Formula (I) is that wherein X₂ is oxygen. The preferred X₃ group for Formula (I) is that wherein X₃ is hydrogen. Preferred R₂ groups, where applicable, is a C₁₋₂ alkyl optionally substituted by 1 or more halogens. The halogen atoms are preferably fluorine and chlorine, more preferably fluorine. More preferred R₂ groups are those wherein R₂ is methyl, or the fluoro-substituted alkyls, specifically a C₁₋₂ alkyl, such as a -CF₃, -CHF₂, or -CH₂CHF₂ moiety. Most preferred are the -CHF₂ and -CH₃ moieties.

Preferred R₃ moieties are C(O)NH₂, C≡CR₈, CN, C(Z')H, CH₂OH, CH₂F, CF₂H, and CF₃. More preferred are C≡CH and CN. Z' is preferably O or NOR₈.

Preferred R₇ moieties include optionally substituted -(CH₂)₁₋₂(cyclopropyl),-(CH₂)₀₋₂(cyclobutyl), -(CH₂)₀₋₂(cyclopentyl), -(CH₂)₀₋₂(cyclohexyl), -(CH₂)₀₋₂(2-, 3-or 4-pyridyl), (CH₂)₁₋₂(2-imidazolyl), (CH₂)₂(4-morpholinyl), (CH₂)₂(4-piperazinyl), (CH₂)₁₋₂(2-thienyl), (CH₂)₁₋₂(4-thiazolyl), and (CH₂)_{0-2phenyl;}

Preferred rings when R₁₀ and R₁₁ in the moiety -NR₁₀R₁₁ together with the nitrogen to which they are attached form a 5 to 7 membered ring optionally containing at least one additional heteroatom selected from O, N, or S include, but are not limited to 1-imidazolyl, 2-(R₈)-1-imidazolyl, 1-pyrazolyl, 3-(R₈)-1-pyrazolyl, 1-triazolyl, 2-triazolyl, 5-(R₈)-1-triazolyl, 5-(R₈)-2-triazolyl, 5-(R₈)-1-tetrazolyl, 5-(R₈)-2-tetrazolyl, 1-tetrazolyl, 2-tetrazloyl, morpholinyl, piperazinyl, 4-(R₈)-1-piperazinyl, or pyrrolyl ring.

Preferred rings when R₈ and R₁₄ in the moiety -NR₈R₁₄ together with the nitrogen to which they are attached may form a 5 to 7 membered ring optionally containing at least one additional heteroatom selected from O, N, or S include, but are not limited to 1-imidazolyl, 1-pyrazolyl, 1-triazolyl, 2-triazolyl, 1-tetrazolyl, 2-tetrazolyl, morpholinyl, piperazinyl, and pyrrolyl. The respective rings may be additionally substituted, where applicable, on an available nitrogen or carbon by the moiety R₇ as described herein for Formula (I). Illustrations of such carbon substitutions includes, but is not limited to, 2-(R₇)-1-imidazolyl, 4-(R₇)-1-imidazolyl, 5-(R₇)-1-imidazolyl, 3-(R₇)-1-pyrazolyl, 4-(R₇)-1-pyrazolyl, 5-(R₇)-1-pyrazolyl, 4-(R₇)-2-triazolyl, 5-(R₇)-2-triazolyl, 4-(R₇)-1-triazolyl, 5-(R₇)-1-triazolyl, 5-(R₇)-1-tetrazolyl, and 5-(R₇)-2-tetrazolyl. Applicable nitrogen substitution by R₇ includes, but is not limited to, 1-(R₇)-2-tetrazolyl, 2-(R₇)-1-tetrazolyl, 4-(R₇)-1-piperazinyl. Where applicable, the ring may be substituted one or more times by R₇.

Preferred groups for NR₈R₁₄ which contain a heterocyclic ring are 5-(R₁₄)-1-tetrazolyl, 2-(R₁₄)-1-imidazolyl, 5-(R₁₄)-2-tetrazolyl, 4-(R₁₄)-1-piperazinyl, or 4-(R₁₅)-1-piperazinyl.

Preferred rings for R₁₃ include (2-, 4- or 5-imidazolyl), (3-, 4- or 5-pyrazolyl), (4-or 5-triazolyl[1,2,3]), (3- or 5-triazolyl[1,2,4]), (5-tetrazolyl), (2-, 4- or 5-oxazolyl), (3-, 4- or 5-isoxazolyl), (3- or 5-oxadiazolyl[1,2,4]), (2-oxadiazolyl[1,3,4]), (2-thiadiazolyl[1,3,4]), (2-, 4-, or 5-thiazolyl), (2-, 4-, or 5-oxazolidinyl), (2-, 4-, or 5-thiazolidinyl), or (2-, 4-, or 5-imidazolidinyl).

When the R₇ group is optionally substituted by a heterocyclic ring such as imidazolyl, pyrazolyl, triazolyl, tetrazolyl, or thiazolyl, the heterocyclic ring itself may be optionally substituted by R₈ either on an available nitrogen or carbon atom, such as 1-(R₈)-2-imidazolyl, 1-(R₈)-4-imidazolyl, 1-(R₈)-5-imidazolyl, 1-(R₈)-3-pyrazolyl, 1-(R₈)-4-pyrazolyl, 1-(R₈)-5-pyrazolyl, 1-(R₈)-4-triazolyl, or 1-(R₈)-5-triazolyl. Where applicable, the ring may be substituted one or more times by R₈.

Preferred are those compounds of Formula (I) wherein R₁ is -CH₂-cyclopropyl,-CH₂-C₅₋₆ cycloalkyl, -C₄₋₆ cycloalkyl, tetrahydrofuran-3-yl, (3- or 4-cyclopentenyl), benzyl or -C₁₋₂ alkyl optionally substituted by 1 or more fluorines, and -(CH₂)₂₋₄ OH; R₂ is methyl or fluoro-substituted alkyl, R₃ is CN or C≡CR₈, and X is YR₂.

Most preferred are those compounds wherein R₁ is -CH₂-cyclopropyl, cyclopentyl, methyl or CF₂H; R₃ is CN or C≡CH; X is YR₂; Y is oxygen; X₂ is oxygen; X₃ is hydrogen; and R₂ is CF₂H or methyl.

A preferred subgenus of Formula (I) are the compounds of Formula (Ia) 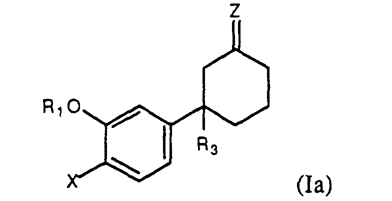 wherein:
R₁ is CH₂-cyclopropyl, CH₂-C₅₋₆ cycloalkyl, C₄₋₆ cycloalkyl, C₇₋₁₁ polycycloalkyl, (3- or 4-cyclopentenyl), phenyl, tetrahydrofuran-3-yl, benzyl or C₁₋₂ alkyl optionally substituted by 1 or more fluorines, -(CH₂)₁₋₃C(O)O(CH₂)₀₋₂CH₃, -(CH₂)₁₋₃O(CH₂)₀₋₂CH₃, and -(CH₂)₂₋₄OH;
X is YR₂, halogen, nitro, NR₄R₅, or formyl amine;
Y is O or S(O)_{m'};
m' is 0, 1, or 2;
R₂ is -CH₃ or -CH₂CH₃ optionally substituted by 1 or more halogens;
R₃ is C₁₋₄ alkyl, halo-substituted C₁₋₄ alkyl, CH₂NHC(O)C(O)NH₂, CN, CH₂OR₈, C(Z')H, C(O)OR₈, C(O)NR₈R₁₀, or C≡CR₈;
Z' is O or NOR₈;
Z is O, NR₇, NOR₁₄, NOR₁₅, NNR₄R₁₄, NNR₄R₁₅, NCN, C(CN)₂, C(-CN)OC(O)R₉, C(-CN)OR₉, CR₁₄C(O)OR₈, or =Z is 2-(1,3-dithiane), dimethylthio ketal, 2-(1,3-dioxolane), or dimethyl ketal;
R₇ is -(CR₄R₅)_{q}R₁₂ or C₁₋₆ alkyl wherein the R₁₂ or C₁₋₆ alkyl group is optionally substituted one or more times by C₁₋₂ alkyl optionally substituted by one to three fluorines, -F, -Br, -Cl, -NO₂, -Si(R₄)₃, -NR₁₀R₁₁, -C(O)R₈, -CO₂R₈, -OR₈, -CN, -C(O)NR₁₀R₁₁, -OC(O)NR₁₀R₁₁, -OC(O)R₈, -NR₁₀C(O)NR₁₀R₁₁, -NR₁₀C(O)R₁₁, -NR₁₀C(O)OR₉, -NR₁₀C(O)R₁₃, -C(NR₁₀)NR₁₀R₁₁, -C(NCN)NR₁₀R₁₁,-C(NCN)SR₉, -NR₁₀C(NCN)SR₉, -NR₁₀C(NCN)NR₁₀R₁₁, -NR₁₀S(O)₂R₉, -S(O)_{m'}R₉, -NR₁₀C(O)C(O)NR₁₀R₁₁, -NR₁₀C(O)C(O)R₁₀, thiazolyl, imidazolyl, oxazolyl, pyrazolyl, triazolyl, or tetrazolyl;
q is 0, 1, or 2;
R₁₂ is C₃₋₇ cycloalkyl, (2-, 3- or 4-pyridyl), (1- or 2-imidazolyl), piperazinyl, morpholinyl, (2- or 3-thienyl), (4- or 5-thiazolyl), or phenyl;
R₈ is independently selected from hydrogen or R₉;
R₉ is C₁₋₄ alkyl optionally substituted by one to three fluorines;
R₁₀ is OR₈ or R₁₁;
R₁₁ is hydrogen or C₁₋₄ alkyl optionally substituted by one to three fluorines; or when R₁₀ and R₁₁ are as NR₁₀R₁₁ they may together with the nitrogen form a 5 to 7 membered ring optionally containing at least one additional heteroatom selected from O, N, or S;
R₁₃ is oxazolidinyl, oxazolyl, thiazolyl, pyrazolyl, triazolyl, tetrazolyl, imidazolyl, imidazolidinyl, thiazolidinyl, isoxazolyl, oxadiazolyl, or thiadiazolyl, and each of these heterocyclic rings is connected through a carbon atom and each may be unsubstituted or substituted by one or two C₁₋₂ alkyl groups;
R₁₄ is hydrogen or R₇; or when R₈ and R₁₄ are as NR₈R₁₄ they may together with the nitrogen form a 5 to 7 membered ring optionally containing one or more additional heteroatoms selected from O, N, or S;
R₁₅ is C(O)R₁₄, C(O)NR₄R₁₄, S(O)₂R₇, or S(O)₂NR₄R₁₄; provided that when R₁₂ is N-imidazolyl, N-triazolyl, N-pyrrolyl, N-piperazinyl, or N-morpholinyl, then q is not 1;
or the pharmaceutically acceptable salts thereof.

An exemplified preferred compound of Formula (I) is 3-cyano-3-(cyclopentyloxy-4-methoxyphenyl)cyclohexan-1-one.

It will be recognized that some of the compounds of Formula (I) may exist in both racemic and optically active forms; some may also exist in distinct diastereomeric forms possessing distinct physical and biological properties. All of these compounds are considered to be within the scope of the present invention.

The term "C₁₋₃ alkyl", "C₁₋₄ alkyl", "C₁₋₆ alkyl" or "alkyl" groups as used herein is meant to include both straight or branched chain radicals of 1 to 10, unless the chain length is limited thereto, including, but not limited to methyl, ethyl, n-propyl, isopropyl, n-butyl, sec-butyl, isobutyl, *tert*-butyl, and the like.

"Alkenyl" means both straight or branched chain radicals of 1 to 6 carbon lengths, unless the chain length is limited thereto, including but not limited to vinyl, 1-propenyl, 2-propenyl, 2-propynyl, or 3-methyl-2-propenyl.

The term "cycloalkyl" or "cycloalkyl alkyl" means groups of 3-7 carbon atoms, such as cyclopropyl, cyclopropylmethyl, cyclopentyl, or cyclohexyl.

"Aryl" or "aralkyl", unless specified otherwise, means an aromatic ring or ring system of 6-10 carbon atoms, such as phenyl, benzyl, phenethyl, or naphthyl. Preferably the aryl is monocyclic, i.e, phenyl. The alkyl chain is meant to include both straight or branched chain radicals of 1 to 4 carbon atoms.

"Heteroaryl" means an aromatic ring system containing one or more heteroatoms, such as imidazolyl, triazolyl, oxazolyl, pyridyl, pyrimidyl, pyrazolyl, pyrrolyl, furanyl, or thienyl.

"Halo" means all halogens, i.e., chloro, fluoro, bromo, or iodo.

"Inhibiting the production of IL-1" or "inhibiting the production of TNF" means:
a) a decrease of excessive *in vivo* IL-1 or TNF levels, respectively, in a human to normal levels or below normal levels by inhibition of the *in vivo* release of IL-1 by all cells, including but not limited to monocytes or macrophages;
b) a down regulation, at the translational or transcriptional level, of excessive *in vivo* IL-1 or TNF levels, respectively, in a human to normal levels or below normal levels; or
c) a down regulation, by inhibition of the direct synthesis of IL-1 or TNF levels as a postranslational event.

The phrase "TNF mediated disease or disease states" means any and all disease states in which TNF plays a role, either by production of TNF itself, or by TNF causing another cytokine to be released, such as but not limited to IL-1 or IL-6. A disease state in which IL-1, for instance is a major component, and whose production or action, is exacerbated or secreted in response to TNF, would therefore be considered a disease state mediated by TNF. As TNF-β (also known as lymphotoxin) has close structural homology with TNF-α (also known as cachectin), and since each induces similar biologic responses and binds to the same cellular receptor, both TNF-α and TNF-β are inhibited by the compounds of the present invention and thus are herein referred to collectively as "TNF" unless specifically delineated otherwise. Preferably TNF-α is inhibited.

"Cytokine" means any secreted polypeptide that affects the functions of cells, and is a molecule which modulates interactions between cells in immune, inflammatory, or hematopoietic responses. A cytokine includes, but is not limited to, monokines and lymphokines regardless of which cells produce them.
The cytokine inhibited by the present invention for use in the treatment of a HIV-infected human must be a cytokine which is implicated in (a) the initiation and/or maintenance of T cell activation and/or activated T cell-mediated HIV gene expression and/or replication, and/or (b) any cytokine-mediated disease associated problem such as cachexia or muscle degeneration. Preferrably, his cytokine is TNF-α.

All of the compounds of Formula (I) are useful in the method of inhibiting the production of TNF, preferably by macrophages, monocytes or macrophages and monocytes, in a mammal, including humans, in need thereof. All of the compounds of Formula (I) are useful in the method of inhibiting or mediating the enzymatic or catalytic activity of PDE IV and in treatment of disease states mediated thereby.

### METHODS OF PREPARATION

Preparing compounds of Formula (I) can be carried out by one of skill in the art according to the procedures outlined in the Examples, *infra*. The preparation of any remaining compounds of Formula (I) not described therein may be prepared by the analogous processes disclosed herein which comprise:
a) for compounds wherein X and X₃ are other than Br, I, NO₂, amine, formyl amine, or S(O)m' when m' is 1 or 2, reacting a compound of Formula (2) 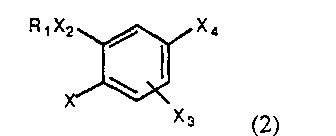
   wherein R₁ represents R₁ as defined in relation to Formula (I) or a group convertable to R₁ and X represents X as defined in relation to Formula (I) or a group convertable to X and X₃ represents X₃ as defined in relation to Formula (I) or a group convertable to X₃ and X₄ is a counter ion (e.g., lithium, magnesium, etc.) with a compound of the Formula (3) 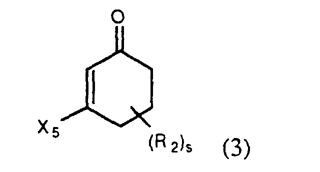
   wherein X₅ is, e.g., OCH₃, OC₂H₅, OCH(CH₃)₂, etc., followed by appropriate workup to provide a compound of the Formula (4) 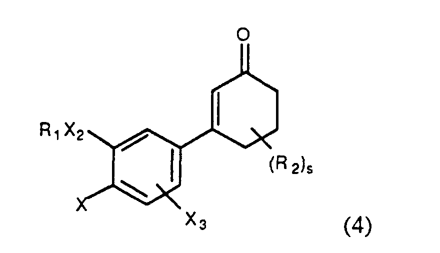
   wherein R₁ represents R₁ as defined in relation to Formula (I) or a group convertable to R₁ and X represents X as defined in relation to Formula (I) or a group convertable to X and X₃ represents X₃ as defined in relation to Formula (I) or a group convertable to X₃ (see the patent application WO 9115-451-A published by WIPO). Michael-type reaction of such a compound of the Formula (4) with the appropriate precursor of R₃ then provides a compound of the Formula (1); for example, use of diethylaluminum cyanide provides a compound of the Formula (1) wherein R₁ represents R₁ as defined in relation to Formula (I) or a group convertable to R₁ and X represents X as defined in relation to Formula (I) or a group convertable to X and X₃ represents X₃ as defined in relation to Formula (I) or a group convertable to X₃ and R₃ is CN.
      Compounds of Formula (I) wherein R₃ is CHO and Z is O may be prepared from the compound of Formula (I) in which R₃ is CN and Z is O after appropriate protection of the ketone as, e.g., a ketal, followed by reduction of the CN moiety with, e.g., di-isobutylaluminum hydride, followed by appropriate workup and kedif Formula (I) wherein R₃ is CH₂OH and Z is O may be prepared by reduction of the compound of Formula (I) in which R₃ is CHO and =Z is a ketal protecting group with, e.g., sodium borohydride, followed by appropriate workup and ketone deprotection.
      Compounds of Formula (I) wherein R₃ is CH₂NR₈R₈ and Z is O may be prepared by reduction of the compound of Formula (I) in which R₃ is CN and =Z is a ketal protecting group with, e.g., lithium aluminum hydride or hydrogen in the presence of a catalyst, followed by appropriate workup, standard alkylation by R₈ and then ketone deprotection.
      Compounds of Formula (I) wherein R₃ is C≡CR_{8'} and Z is O may be prepared from the compound of Formula (I) in which R₃ is CHO and =Z is a ketal protecting group by reaction with a mixture of dimethyl (diazomethyl)phosphonate and potassium *t*-butoxide or other suitable base, in an inert solvent, such as tetrahydrofuran, at reduced temperature, followed by appropriate workup and ketone deprotection to provide the compounds of Formula (I) wherein R₃ is C≡CH; alternatively, prior to ketone deprotection, alkylation of the acetylene under the appropriate conditions with a strong base followed by an alkylating agent, R₈L, wherein L is a leaving group and R_{8'} is not H, followed by ketone deprotection, provides compounds of Formula (I) wherein R₃ is C≡ CR_{8'}.
      Compounds of Formula (I) wherein R₃ is CH₂F and Z is O may be prepared from the compound of Formula (I) wherein R₃ is CH₂OH and =Z is a ketal protecting group by treatment with diethyl-aminosulfur trifluoride (DAST) followed by ketone deprotection.
      Compounds of Formula (I) wherein R₃ is CHF₂ and Z is O may be prepared from the compound of Formula (I) wherein R₃ is CHO and =Z is a ketal protecting group by treatment with diethylaminosulfur trifluoride (DAST) followed by ketone deprotection.
      The compounds of Formula (I) where R₃ is C₁ alkyl and Z is O may be prepared from the compound of Formula (I) wherein R₃ is CH₂OH and =Z is a protected ketone by reductive removal of the alcohol with lithium in ammonia, with aluminum hydride, or by conversion of the alcohol to the corresponding thiocarbamate followed by reduction with, e.g., tributyltin hydride or trialkylsilyl hydride, and ketone deprotection; alternatively, the compounds of Formula (I) wherein R₃ is C₁ alkyl and Z is O may be prepared from the compound of Formula (I) wherein R₃ is CHO and =Z is a protected ketone by thioketal formation, desulfurization and ketal deprotection.
      Compounds of Formula (I) where R₃ is C₂₋₄ alkyl or halogen substituted C₂₋₄ alkyl and Z is O may be prepared by analogous deoxygenation procedures from the corresponding alcohol derived from reaction of the compound of Formula (I) wherein R₃ is CHO and =Z is a protected ketone with a metal alkyl or a halogen substituted C₂₋₄ metal alkyl reagent and subsequent deprotection to liberate the =Z ketone.
      Compounds of Formula (I) wherein R₃ is vinyl and Z is O may be prepared by, e.g., Wittig or other olefination reaction of the compound of Formula (I) wherein R₃ is CHO and =Z is a protected ketone, and subsequent deprotection to liberate the =Z ketone.
      Compounds of Formula (I) wherein R₃ is cyclopropyl and Z is O may be prepared from the compound of Formula (I) wherein R₃ is vinyl and =Z is a protected ketone by reaction with, e.g., methylene iodide and zinc-copper couple, with subsequent deprotection to liberate the =Z ketone.
      Most compounds of Formula (I) wherein Z is not O are prepared from the corresponding compounds of Formula (I) wherein Z is O by reaction with the appropriate amine, alcohol, active methylene reagent, thiol, etc., in the presence of a catalyst or with removal of water, if required, as described in United States patent application 07/862,083 filed 2 April 1992 and its progeny USSN 07/968,753 filed 30 October 1992; however, when R₃ is CHO, this R₃ group may require protection as, e.g., a ketal, during reaction followed by deprotection.
b) Compounds of Formula (I) wherein X or X₃ is formyl amine and Z is O may be prepared by formylating, at the last step, a compound wherein =Z is a protected ketone and X is NH₂, obtained by removal of a protecting group from the amine functionality; such protective groups are well known to those skilled in the art, See Greene, T. and Wuts, P.G.M., Protecting Groups in Organic Synthesis, 2nd Ed., John Wiley and Sons, New York (1991).
c) Compounds of Formula (I) wherein X or X₃ is Br or I and Z is O may be prepared from a similarly deprotected amine by diazotization of the amine and diazonium displacement via Sandmeyer reaction.
d) Compounds of Formula (I) wherein X or X₃ is NO₂ and Z is O may be prepared from a similarly deprotected amine by oxidation of the amine to the nitro group.
e) Compounds of Formula (I) wherein Y is S(O)m' when m' is 1 or 2 and Z is O may be prepared from the compounds of Formula (I) wherein Y is S by oxidation of the SR₂ moiety under conditions well known to those skilled in the art.

The following examples are set out to illustrate how to make the compounds of this invention and methods for determining associated therapeutic activity. These examples are not intended to limit the invention in any manner, their purpose is illustrative rather than limiting.

### EXAMPLE 1

### Preparation of 3-cyano-3-(cyclopentyloxy-4-methoxyphenyl)cyclohexan-1-one

### 1a. 3-(3-cyclopentyloxy-4-methoxyphenyl)cyclohex-2-en-1-one

n-Butyllithium (2.5 M in hexanes, 15.5 mL, 38.9 mmol) was added dropwise over 30 min to a solution of 3-cyclopentyloxy-4-methoxybromobenzene (10 g, 37 mmol,) in dry tetrahydrofuran (100 mL) at -78°C under an argon atmosphere. After 1.5 h, this solution was cannulated into a solution of 3-methoxycyclohex-2-enone (4.62g, 37.4 mmol, prepared as in Pearson, A.J.; Richards, I.C.; Gardner, D.V. J. Org. Chem. 1984, 49, 3887-3891) in dry tetrahydrofuran (50 mL) at 0°C under an argon atmosphere. After 2 h at room temperature, a mixture of ether and water was added, the aqueous layer was extracted three times with ether, the combined extract was washed with water and brine, was dried (magnesium sulfate) and was evaporated. Trituration from ether/hexanes provided an off-white solid (7.33 g, 68%). Further purification of the mother liquor by flash chromatography, eluting with 1:3 ethyl acetate/hexanes, followed by trituration from ether/hexanes, provided a white solid (1.59 g, 7%). mp 89-90°C. Anal. (C₁₈H₂₂O₃·1/8 H₂O) calcd: C 74.91, H 7.77; found: C 74 96, H 7.76.

### 1b 3-cyano-3-(cyclopentyloxy-4-methoxyphenyl)cyclohexan-1-one

To a solution of 3-(3-cyclopentyloxy-4-methoxyphenyl)cyclohex-2-en-1-one (1.46 g, 5.10 mmol) in dry toluene (45 mL) at room temperature under an argon atmosphere was added over 5 min diethylaluminumcyanide (1.0 M solution in toluene, 15.5 mL, 15.5 mmol). After 6 h, the reaction was carefully quenched with sodium hydroxide (2 N, 75 mL, 150 mmol), was extracted three times with methylene chloride, the extract was dried (magnesium sulfate) and was evaporated. Purification by flash chromatography, eluting with 1:4 ethyl acetate/hexanes, provided a pale yellow solid (1.20 g, 75%). mp 110-111°C. Anal. (C₁₉H₂₃NO₃·1/8 H₂0) calcd: C 72.30, H 7.42, N 4.44; found: C 72.24, H 7.45, N 4.58.

### METHODS OF TREATMENT

In order to use a compound of Formula (I) or a pharmaceutically acceptable salt thereof for the treatment of humans and other mammals, it is normally formulated in accordance with standard pharmaceutical practice as a pharmaceutical composition.

The compounds of Formula (I), or a pharmaceutically acceptable salt thereof can be used in the manufacture of a medicament for the prophylatic or therapeutic treatment of any disease state in a human or other mammal which is mediated by inhibition of PDE IV, such as but not limited to asthma, allergic, or inflammatory diseases. The compounds of Formula (I) are administered in an amount sufficient to treat such a disease in a human or other mammal.

For the purposes herein all methods of treatment and dosage regimens apply equally to both the compounds of Formula (I).

In order to use a compound of Formula (I), or a pharmaceutically acceptable salt thereof for the treatment of humans and other mammals, it is normally formulated in accordance with standard pharmaceutical practice as a pharmaceutical composition.

The amount of a compound of Formula (I) required for therapeutic effect on topical administration will, of course, vary with the compound chosen, the nature and severity of the condition and the animal undergoing treatment, and is ultimately at the discretion of the physician.

The daily dosage regimen for oral administration is suitably about .001 mg/kg to 100mg/kg, preferably 0.01 mg/Kg to 40 mg/Kg, of a compound of Formula (I) or a pharmaceutically acceptable salt thereof calculated as the free base. The active ingredient may be administered from 1 to 6 times a day, sufficient to exhibit activity. No toxic effects are expected when these compounds are administered in accordance with the present invention.

### UTILITY EXAMPLES

### EXAMPLE A

### Inhibitory effect of compounds of Formula (I) on in vitro TNF production by human monocytes

The inhibitory effect of compounds of Formula (I) on *in vitro* TNF production by human monocytes may be determined by the protocol as described in Badger *et al*., EPO published Application 0 411 754 A2, February 6, 1991, and in Hanna, WO 90/15534, December 27, 1990.

### EXAMPLE B

Two models of endotoxic shock have been utilized to determine *in vivo* TNF activity for the compounds of Formula (I). The protocol used in these models is described in Badger *et al*., EPO published Application 0411 754 A2, February 6, 1991, and in Hanna, WO 90/15534, December 27, 1990.

The compound of Example 1 herein demonstrated a positive *in vivo* response in reducing serum levels of TNF induced by the injection of endotoxin.

### EXAMPLE C

### Isolation of PDE Isozymes

The phosphodiesterase inhibitory activity and selectivity of the compounds of Formula (I) can be determined using a battery of five distinct PDE isozymes. The tissues used as sources of the different isozymes are as follows: 1) PDE Ib, porcine aorta; 2) PDE Ic, guinea-pig heart; 3) PDE III, guinea-pig heart; 4) PDE IV, human monocyte; and 5) PDE V (also called "Ia"), canine trachealis. PDEs Ia, Ib, Ic and III are partially purified using standard chromatographic techniques [Torphy and Cieslinski, Mol. Pharmacol., 37:206-214, 1990]. PDE IV is purified to kinetic homogeneity by the sequential use of anion-exchange followed by heparin-Sepharose chromatography [Torphy *et al*., J. Biol. Chem., 267:1798-1804, 1992].

Phosphodiesterase activity is assayed as described in the protocol of Torphy and Cieslinski, Mol. Pharmacol., 37:206-214, 1990. Positive IC₅₀'s in the nanomolar to µM range for compounds of the workings examples described herein for Formula (I) have been demonstrated.

### EXAMPLE D

The ability of selected PDE IV inhibitors to increase cAMP accumulation in intact tissues is assessed using U-937 cells, a human monocyte cell line that has been shown to contain a large amount of PDE IV. To assess the activity of PDE IV inhibition in intact cells, nondifferentiated U-937 cells (approximately 10⁵ cells/reaction tube) were incubated with various concentrations (0.01-1000 µM) of PDE inhibitors for one minute and 1µM prostaglandin E2 for an additional four minutes. Five minutes after initiating the reaction, cells were lysed by the addition of 17.5% perchloric acid, the pH was neutralized by the addition of 1M potassium carbonate and cAMP content was assessed by RIA. A general protocol for this assay is described in Brooker *et al*., Radioimmunassay of cyclic AMP and cyclic GMP., Adv. Cyclic Nucleotide Res., 10:1-33, 1979. The compounds of the working examples as described herein for Formula (I) have demonstrated a positive EC₅₀s in the µM range in the above assay.

## Claims

1. A compound of formula (I): 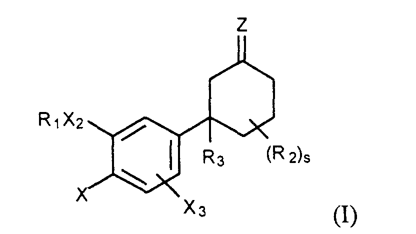 wherein:
R₁ is -(CR₄R₅)ₙC(O)O(CR₄R₅)ₘR₆, -(CR₄R₅)ₙC(O)NR₄(CR₄R₅)ₘR₆,-(CR₄R₅)ₙO(CR₄R₅)ₘR₆, or -(CR₄R₅)ᵣR₆ wherein the alkyl moieties may be optionally substituted with one or more halogens;
m is 0 to 2;
n is 1 to 4;
r is 0 to 6;
R₄ and R₅ are independently selected hydrogen or C₁₋₂ alkyl;
R₆ is hydrogen, methyl, hydroxyl, aryl, halo substituted aryl, aryloxyC₁₋₃ alkyl, halo substituted aryloxyC₁₋₃ alkyl, indanyl, indenyl, C₇₋₁₁ polycycloalkyl, tetrahydrofuranyl, furanyl, tetrahydropyranyl, pyranyl, tetrahydrothienyl, thienyl, tetrahydrothiopyranyl, thiopyranyl, C₃₋₆ cycloalkyl, or a C₄₋₆ cycloalkyl containing one or two unsaturated bonds, wherein the cycloalkyl and heterocyclic moieties may be optionally substituted by 1 to 3 methyl groups or one ethyl group;
provided that:
a) when R₆ is hydroxyl, then m is 2; or
b) when R₆ is hydroxyl, then r is 2 to 6; or
c) when R₆ is 2-tetrahydropyranyl, 2-tetrahydrothiopyranyl, 2-tetrahydrofuranyl, or 2-tetrahydrothienyl, then m is 1 or 2; or
d) when R₆ is 2-tetrahydropyranyl, 2-tetrahydrothiopyranyl, 2-tetrahydrofuranyl, or 2-tetrahydrothienyl, then r is 1 to 6;
e) when n is 1 and m is 0, then R₆ is other than H in -(CR₄R₅)ₙO(CR₄R₅)ₘR₆;
X is YR₂, halogen, nitro, NR₄R₅, or formyl amine;
Y is O or S(O)_{m'};
m' is 0, 1, or 2;
X₂ is O or NR₈;
X₃ is hydrogen or X;
R₂ is independently selected from -CH₃ or -CH₂CH₃ optionally substituted by 1 or more halogens;
s is 0 to 4;
R₃ is C₁₋₄ alkyl, fluoro-substituted C₁₋₄ alkyl, CH₂NHC(O)C(O)NH₂,-CH=CR_{8'}R_{8'}, cyclopropyl optionally substituted by R_{8'}, CN, CH₂OR₈, CH₂NR₈R₁₀, C(Z')H, C(O)OR₈, C(O)NR₈R₁₀, or C≡CR_{8'};
Z' is O, NR₉, NOR₈, NCN, C(-CN)₂, CR₈CN, CR₈NO₂, CR₈C(O)OR₈, CR₈C(O)NR₈R₈, C(-CN)NO₂, C(-CN)C(O)OR₉, or C(-CN)C(O)NR₈R₈ ;
Z is O, NR₇, NCR₄R₅C₂₋₆ alkenyl, NOR₁₄, NOR₁₅, NOCR₄R₅C₂₋₆ alkenyl, NNR₄R₁₄, NNR₄R₁₅, NCN, NNR₈C(O)NR₈R₁₄, NNR₈C(S)NR₈R₁₄, C(-CN)₂, CR₁₄CN, CR₁₄C(O)OR₈, CR₁₄C(O)NR₈R₁₄, C(-CN)NO₂, C(-CN)C(O)OR₉, C(-CN)OC(O)R₉, C(-CN)OR₉, C(-CN)C(O)NR₈R₁₄, or =Z is 2-(1,3-dithiane), 2-(1,3-dithiolane), dimethylthio ketal, diethylthio ketal, 2-(1,3-dioxolane), 2(1,3-dioxane), 2-(1,3-oxathiolane), dimethyl ketal or diethyl ketal;
R₇ is -(CR₄R₅)_{q}R₁₂ or C₁₋₆ alkyl wherein the R₁₂ or C₁₋₆ alkyl group is optionally substituted one or more times by C₁₋₂ alkyl optionally substituted by one to three fluorines,-F, -Br, -Cl, -NO₂, -Si(R₄)₃, -NR₁₀R₁₁, -C(O)R₈, -CO₂R₈, -OR₈, -CN, -C(O)NR₁₀R₁₁,-OC(O)NR₁₀R₁₁, -OC(O)R₈, -NR₁₀C(O)NR₁₀R₁₁, -NR₁₀C(O)R₁₁, -NR₁₀C(O)OR₉, -NR₁₀C(O)R₁₃, -C(NR₁₀)NR₁₀R₁₁, -C(NCN)NR₁₀R₁₁, -C(NCN)SR₉, -NR₁₀C(NCN)SR₉, -NR₁₀C(NCN)NR₁₀R₁₁, -NR₁₀S(O)₂R₉, -S(O)_{m'}R₉, -NR₁₀C(O)C(O)NR₁₀R₁₁, -NR₁₀C(O)C(O)R₁₀, thiazolyl, imidazolyl, oxazolyl, pyrazolyl, triazolyl, or tetrazolyl;
q is 0, 1, or 2;
R₁₂ is C₃₋₇ cycloalkyl, (2-, 3- or 4-pyridyl), pyrimidyl, pyrazolyl, (1- or 2-imidazolyl), thiazolyl, triazolyl, pyrrolyl, piperazinyl, piperidinyl, morpholinyl, furanyl, (2-or 3-thienyl), (4- or 5-thiazolyl), quinolinyl, naphthyl, or phenyl;
R₈ is independently selected from hydrogen or R₉;
R_{8'} is R₈ or fluorine;
R₉ is C₁₋₄ alkyl optionally substituted by one to three fluorines;
R₁₀ is OR₈ or R₁₁;
R₁₁ is hydrogen, or C₁₋₄ alkyl optionally substituted by one to three fluorines; or when R₁₀ and R₁₁ are as NR₁₀R₁₁ they may together with the nitrogen form a 5 to 7 membered ring optionally containing at least one additional heteroatom selected from O, N, or S;
R₁₃ is oxazolidinyl, oxazolyl, thiazolyl, pyrazolyl, triazolyl, tetrazolyl, imidazolyl, imidazolidinyl, thiazolidinyl, isoxazolyl, oxadiazolyl, or thiadiazolyl, and each of these heterocyclic rings is connected through a carbon atom and each may be unsubstituted or substituted by one or two C₁₋₂ alkyl groups;
R₁₄ is hydrogen or R₇; or when R₈ and R₁₄ are as NR₈R₁₄ they may together with the nitrogen form a 5 to 7 membered ring optionally containing one or more additional heteroatoms selected from O, N, or S;
R₁₅ is C(O)R₁₄, C(O)NR₄R₁₄, S(O)₂R₇, or S(O)₂NR₄R₁₄;
provided that:
(f) when Z is O, X₂ is oxygen, X₃ is hydrogen, s is 0, and X is YR₂, then R₃ is other than hydrogen;
(g) when R₁₂ is N-pyrazolyl, N-imidazolyl, N-triazolyl, N-pyrrolyl, N-piperazinyl, N-piperidinyl, or N-morpholinyl, then q is not 1; or
(h) when Z is O or =Z is 2-(1,3-dioxolane) and R₃ is CH₃, CH₂OH or CH₂OC₁₋₄ alkyl, then R₁X₂ is not C₁-C₃ alkoxy and X is not halogen, methoxy, ethoxy, methylthio. or ethylthio; not being 3-cyano-3-(3,4-dimethoxyphenyl) cyclohexan-1-one;
or pharmaceutically acceptable salts thereof.

2. A compound according to claim 1 wherein 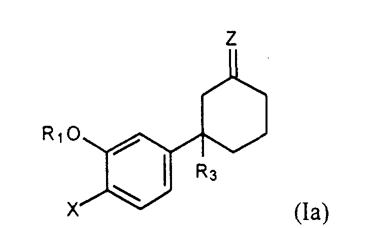 wherein:
R₁ is CH₂-cyclopropyl, CH₂-C₅₋₆ cycloalkyl, C₄₋₆ cycloalkyl, C₇₋₁₁ polycycloalkyl, (3- or 4-cyclopentenyl), phenyl, tetrahydrofuran-3-yl, benzyl or C₁₋₂ alkyl optionally substituted by 1 or more fluorines, -(CH₂)₁₋₃C(O)O(CH₂)₀₋₂CH₃, -(CH₂)₁₋₃O(CH₂)₀₋₂CH₃, and -(CH₂)₂₋₄OH;
X is YR₂, halogen, nitro, NR₄R₅, or formyl amine;
Y is O or S(O)_{m'};
m' is 0, 1, or 2;
R₂ is -CH₃ or -CH₂CH₃ optionally substituted by 1 or more halogens;
R₃ is C₁₋₄ alkyl, halo-substituted C₁₋₄ alkyl, CH₂NHC(O)C(O)NH₂, CN, CH₂OR₈, C(Z')H, C(O)OR₈, C(O)NR₈R₁₀, or C≡CR₈;
Z' is O or NOR₈;
Z is O, NR₇, NOR₁₄, NOR₁₅, NNR₄R₁₄, NNR₄R₁₅, NCN, C(CN)₂, C(-CN)OC(O)R₉, C(-CN)OR₉, CR₁₄C(O)OR₈, or =Z is 2-(1,3-dithiane), dimethylthio ketal, 2-(1,3-dioxolane), or dimethyl ketal;
R₇ is -(CR₄R₅)_{q}R₁₂ or C₁₋₆ alkyl wherein the R₁₂ or C₁₋₆ alkyl group is optionally substituted one or more times by C₁₋₂ alkyl optionally substituted by one to three fluorines,-F, -Br, -Cl, -NO₂, -Si(R₄)₃, -NR₁₀R₁₁, -C(O)R₈, -CO₂R₈, -OR₈, -CN, -C(O)NR₁₀R₁₁,-OC(O)NR₁₀R₁₁, -OC(O)R₈, -NR₁₀C(O)NR₁₀R₁₁, -NR₁₀C(O)R₁₁, -NR₁₀C(O)OR₉, -NR₁₀C(O)R₁₃, -C(NR₁₀)NR₁₀R₁₁, -C(NCN)NR₁₀R₁₁, -C(NCN)SR₉, -NR₁₀C(NCN)SR₉, -NR₁₀C(NCN)NR₁₀R₁₁, -NR₁₀S(O)₂R₉, -S(O)_{m'}R_{9,} -NR₁₀C(O)C(O)NR₁₀R₁₁, -NR₁₀C(O)C(O)R₁₀, thiazolyl, imidazolyl, oxazolyl, pyrazolyl, triazolyl, or tetrazolyl;
q is 0, 1, or 2;
R₁₂ is C₃₋₇ cycloalkyl, (2-, 3- or 4-pyridyl), (1- or 2-imidazolyl), piperazinyl, morpholinyl, (2- or 3-thienyl), (4- or 5-thiazolyl), or phenyl;
R₈ is independently selected from hydrogen or R₉;
R₉ is C₁₋₄ alkyl optionally substituted by one to three fluorines;
R₁₀ is OR₈ or R₁₁;
R₁₁ is hydrogen or C₁₋₄ alkyl optionally substituted by one to three fluorines; or when R₁₀ and R₁₁ are as NR₁₀R₁₁ they may together with the nitrogen form a 5 to 7 membered ring optionally containing at least one additional heteroatom selected from O, N, or S;
R₁₃ is oxazolidinyl, oxazolyl, thiazolyl, pyrazolyl, triazolyl, tetrazolyl, imidazolyl, imidazolidinyl, thiazolidinyl, isoxazolyl, oxadiazolyl, or thiadiazolyl, and each of these heterocyclic rings is connected through a carbon atom and each may be unsubstituted or substituted by one or two C₁₋₂ alkyl groups;
R₁₄ is hydrogen or R₇; or when R₈ and R₁₄ are as NR₈R₁₄ they may together with the nitrogen form a 5 to 7 membered ring optionally containing one or more additional heteroatoms selected from O, N, or S;
R₁₅ is C(O)R₁₄, C(O)NR₄R₁₄, S(O)₂R₇, or S(O)₂NR₄R₁₄;
provided that when R₁₂ is N-imidazolyl, N-triazolyl, N-pyrrolyl, N-piperazinyl, or N-morpholinyl, then q is not 1;
or the pharmaceutically acceptable salts thereof.

3. A compound according to either claim 1 or 2 wherein R₁ is -CH₂-cyclopropyl, -CH₂-C₅₋₆ cycloalkyl, -C₄₋₆ cycloalkyl, tetrahydrofuran-3-yl, (3- or 4-cyclopentenyl), benzyl or -C₁₋₂ alkyl optionally substituted by I or more fluorines, and-(CH₂)₂₋₄ OH; R₂ is methyl or fluoro-substituted alkyl, R₃ is CN or C≡CR₈, and X is YR₂.

4. A compound according to any one of claims 1 - 3 wherein R₁ is -CH₂-cyclopropyl, cyclopentyl, methyl or CF₂H; R₃ is CN or C≡CH; X is YR₂; Y is oxygen; X₂ is oxygen; X₃ is hydrogen; and R₂ is CF₂H or methyl.

5. A compound according to claim 1 which is 3-cyano-3-(cyclopentyloxy-4-methoxyphenyl)cyclohexan-1-one.

6. A pharmaceutical composition comprising a compound of Formula (I) according to any one of claims 1 - 5 and a pharmaceutically acceptable excipient.

7. The use of a compound according to any one of claims 1 - 5 for manufacturing a medicament for use in treating an allergic or inflammatory state.

8. The use as described in claim 7 wherein the manufactured medicament is used for treating asthma.

## Patentansprüche

1. Verbindung der Formel (I): 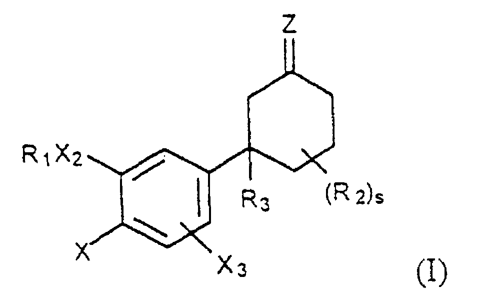
wobei R¹ die Bedeutung -(CR₄R₅)ₙC(O)O(CR₄R₅)ₘR₆, -(CR₄R₅)ₙC(O)NR₄(CR₄R₅)ₘR₆,-(CR₄R₅)ₙO(CR₄R₅)ₘR₆ oder -(CR₄R₅)ᵣR₆ hat, wobei die Alkyleinheiten gegebenenfalls durch ein oder mehrere Halogenatome substituiert sein können;
m von 0 bis 2 reicht;
n von 1 bis 4 reicht;
r von 0 bis 6 reicht;
R⁴ und R⁵ unabhängig voneinander aus einem Wasserstoffatom oder einem C₁₋₂-Alkylrest ausgewählt sind;
R⁶ ein Wasserstoffatom, eine Methyl-, Hydroxylgruppe, ein Aryl-, halogensubstituierter Aryl-, Aryloxy-C₁₋₃-alkyl-, halogensubstituierter Aryloxy-C₁₋₃-alkylrest, eine Indanyl-, Indenylgruppe, ein C₇₋₁₁-Polycycloalkylrest, eine Tetrahydrofuranyl-, Furanyl-, Tetrahydropyranyl-, Pyranyl-, Tetrahydrothienyl-, Thienyl-, Tetrahydrothiopyranyl-, Thiopyranylgruppe, ein C₃₋₆-Cycloalkyl- oder ein C₄₋₆-Cycloalkylrest ist, der ein oder zwei ungesättigte Bindungen enthält, wobei die Cycloalkyl- und heterocyclischen Einheiten gegebenenfalls durch 1 bis 3 Methylgruppen oder eine Ethylgruppe substituiert sein können;
vorausgesetzt, dass:
a) wenn R₆ eine Hydroxylgruppe ist, m dann gleich 2 ist; oder
b) wenn R₆ eine Hydroxylgruppe ist, r dann gleich 2 bis 6 ist; oder
c) wenn R₆ eine 2-Tetrahydropyranyl-, 2-Tetrahydrothiopyranyl-, 2-Tetrahydrofuranyl- oder 2-Tetrahydrothienyl-Gruppe ist, m dann gleich 1 oder 2 ist; oder
d) wenn R₆ eine 2-Tetrahydropyranyl-, 2-Tetrahydrothiopyranyl-, 2-Tetrahydrofuranyl- oder 2-Tetrahydrothienyl-Gruppe ist, r dann gleich 1 bis 6 ist;
e) wenn n gleich 1 ist und m gleich 0 ist, R₆ dann in -(CR₄R₅)ₙO(CR₄R₅)ₘR₆ eine andere Bedeutung als H hat;
X ein Rest YR₂, ein Halogenatom, eine Nitrogruppe, NR₄R₅ oder Formylamin ist;
Y die Bedeutung O oder S(O)_{m'} hat;
m' gleich 0, 1 oder 2 ist;
X₂ die Bedeutung O oder NR₈ hat;
X₃ ein Wasserstoffatom oder X ist;
R₂ unabhängig aus -CH₃ oder -CH₂CH₃, welche gegebenenfalls durch 1 oder mehrere Halogenatome substituiert sind, ausgewählt ist;
s gleich 0 bis 4 ist;
R₃ ein C₁₋₄-Alkyl-, fluorsubstituierter C₁₋₄-Alkylrest, ein Rest CH₂NHC(O)C(O)NH₂,-CH=CR₈,R_{8'}, eine gegebenenfalls durch R_{8'} substituierte Cyclopropylgruppe, CN, CH₂OR₈, CH₂NR₈R₁₀, C(Z')H, C(O)OR₈, C(O)NR₈R₁₀ oder C≡CR8' ist;
Z' die Bedeutung O, NR₉, NOR₈, NCN, C(-CN)₂ CR₈CN, CR₈NO₂, CR₈C(O)OR₈, CR₈C(O)NR₈R₈, C(-CN)NO₂, C(-CN)C(O)OR₉ oder C(-CN)C(O)NR₈R₈ hat;
Z die Bedeutung O, NR₇, NCR₄R₅C₂₋₆-Alkenyl, NOR₁₄, NOR₁₅, NOCR₄R₅C₂₋₆-Alkenyl, NNR₄R₁₄, NNR₄R₁₅, NCN, NNR₈C(O)NR₈R₁₄, NNR₈C(S)NR₈R₁₄, C(-CN)₂, CR₁₄CN, CR₁₄C(O)OR₈, CR₁₄C(O)NR₈R₁₄, C(-CN)NO₂, C(-CN)C(O)OR₉, C(-CN)OC(O)R₉, C(-CN)OR₉, C(-CN)C(O)NR₈R₁₄ hat, oder =Z eine 2-(1,3-Dithian)-, 2-(1,3-Dithiolan)-, Dimethylthioketal-, Diethylthioketal-, 2-(1,3-Dioxolan)-, 2-(1,3-Dioxan)-, 2-(1,3-Oxathiolan)-, Dimethylketal- oder Diethylketalgruppe ist;
R₇ ein Rest -(CR₄R₅)_{q}R₁₂ oder ein C₁₋₆-Alkylrest ist, wobei der Rest R₁₂ oder der C₁₋₆-Alkylrest gegebenenfalls ein- oder mehrmals mit einem durch ein bis drei Fluoratome substituierten C₁₋₂-Alkylrest, -F, -Br, -Cl, -NO₂, -Si(R₄)₃, -NR₁₀R₁₁, -C(O)R₈, -CO₂R₈, -OR₈, -CN, -C(O)NR₁₀R₁₁,-OC(O)NR₁₀R₁₁, -OC(O)R₈, -NR₁₀C(O)NR₁₀R₁₁, -NR₁₀C(O)R₁₁, -NR₁₀C(O)OR₉, -NR₁₀C(O)R₁₃, -C(NR₁₀)NR₁₀R₁₁, -C(NCN)NR₁₀R₁₁, -C(NCN)SR₉, -NR₁₀C(NCN)SR₉, -NR₁₀C(NCN)NR₁₀R₁₁, -NR₁₀S(O)₂R₉, -S(O)_{m'}R₉, -NR₁₀C(O)C(O)NR₁₀R₁₁, -NR₁₀C(O)C(O)R₁₀, Thiazolyl, Imidazolyl, Oxazolyl, Pyrazolyl, Triazolyl oder Tetrazolyl substituiert ist;
q gleich 0, 1 oder 2 ist;
R₁₂ ein C₃₋₇-Cycloalkylrest, eine (2-, 3- oder 4-Pyridyl-) , Pyrimidyl-, Pyrazolyl-, (1-oder 2-Imidazolyl-), Thiazolyl-, Triazolyl-, Pyrrolyl-, Piperazinyl-, Piperidinyl-, Morpholinyl-, Furanyl-, (2- oder 3-Thienyl-), (4- oder 5-Thiazolyl-), Chinolinyl-, Naphthyl- oder Phenylgruppe ist;
R₈ unabhängig aus Wasserstoff oder R₉ ausgewählt ist;
R_{8'} die Bedeutung R₈ oder Fluor hat;
R₉ ein C₁₋₄-Alkylrest ist, der gegebenenfalls durch ein oder drei Fluoratome substituiert ist;
R₁₀ die Bedeutung OR₈ oder R₁₁ hat;
R₁₁ ein Wasserstoffatom oder ein C₁₋₄-Alkylrest ist, der gegebenenfalls durch ein bis drei Fluoratome substituiert ist; oder wenn R₁₀ und R₁₁ als NR₁₀R₁₁ vorliegen, sie dann gemeinsam mit dem Stickstoffatom einen 5- bis 7-gliedrigen Ring bilden können, der gegebenenfalls mindestens ein zusätzliches aus O, N oder S ausgewähltes Heteroatom, enthält;
R₁₃ eine Oxazolidinyl-, Oxazolyl-, Thiazolyl-, Pyrazolyl-, Triazolyl-, Tetrazolyl-, Imidazolyl-, Imidazolidinyl-, Thiazolidinyl-, Isoxazolyl-, Oxadiazolyl- oder Thiadiazolyl-Gruppe ist, und diese heterocyclischen Ringe jeweils über ein Kohlenstoffatom miteinander verbunden sind und jeweils unsubstituiert oder durch ein oder zwei C₁₋₂-Alkylreste substituiert sein können;
R₁₄ ein Wasserstoffatom oder ein Rest R₇ ist; oder wenn R₈ und R₁₄ als NR₈R₁₄ vorliegen, sie dann zusammen mit dem Stickstoffatom einen 5- bis 7-gliedrigen Ring bilden können, der ein oder mehrere zusätzliche, aus O, N oder S ausgewählte Heteroatome enthält;
R₁₅ die Bedeutung C(O)R₁₄, C(O)NR₄R₁₄, S(O)₂R₇ oder S(O)₂NR₄R₁₄ hat,
vorausgesetzt, dass:
(f) wenn Z die Bedeutung O hat, X₂ ein Sauerstoffatom ist, X₃ ein Wasserstoffatom ist, s gleich 0 ist, und X die Bedeutung YR₂ hat, R₃ dann eine andere Bedeutung als Wasserstoff hat;
(g) wenn R₁₂ eine N-Pyrazolyl-, N-Imidazolyl-, N-Triazolyl-, N-Pyrrolyl, N-Piperazinyl-, N-Piperidinyl- oder N-Morpholinylgruppe ist, q dann nicht gleich 1 ist; oder
(h) wenn Z die Bedeutung O hat oder =Z eine 2-(1,3-Dioxolan)-Gruppe ist, und R₃ eine CH₃-, CH₂OH-Gruppe oder ein CH₂OC₁₋₄-Alkylrest ist, R₁X₂ dann kein C₁₋₃-Alkoxyrest ist und X kein Halogenatom, keine Methoxy-, Ethoxy-, Methylthio- oder Ethylthiogruppe ist; jedoch nicht 3-Cyano-3-(3,4-dimethoxyphenyl)cyclohexan-1-on;
oder pharmazeutisch verträgliche Salze davon.

2. Verbindung nach Anspruch 1: 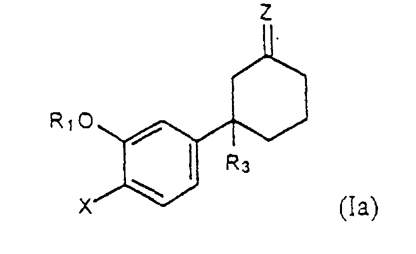 wobei:
R₁ eine CH₂-Cyclopropylgruppe, ein CH₂-C₅₋₆-Cycloalkyl-, C₄₋₆-Cycloalkyl-, C₇₋₁₁-Polycycloalkylrest, eine (3- oder 4-Cyclopentenyl-), Phenyl-, Tetrahydrofuran-3-yl-, Benzylgruppe oder ein C₁₋₂-Alkylrest, der gegebenenfalls durch 1 oder mehrere Fluoratome substituiert ist, -(CH₂)₁₋₃C(O)O(CH₂)₀₋₂CH₃, -(CH₂)₁₋₃O(CH₂)₀₋₂CH₃ und -(CH₂)₂₋₄OH ist;
X ein Rest YR₂, ein Halogenatom, eine Nitrogruppe, NR₄R₅ oder Formylamin ist;
Y die Bedeutung O oder S(O)_{m'} hat;
m' gleich 0, 1 oder 2 ist;
R₂ ein Rest -CH₃ oder -CH₂CH₃ ist, der gegebenenfalls durch 1 oder mehrere Halogenatome substituiert ist;
R₃ ein C₁₋₄-Alkyl-, halogensubstituierter C₁₋₄-Alkyl-Rest, ein Rest CH₂NHC(O)C(O)NH₂, CN, CH₂OR₈, C(Z')H, C(O)OR₈, C(O)NR₈R₁₀ oder C≡CR₈ ist;
Z' die Bedeutung O oder NOR₈ hat;
Z die Bedeutung O, NR₇, NOR₁₄, NOR₁₅, NNR₄R₁₄, NNR₄R₁₅, NCN, C(CN)₂, C(-CN)OC(O)R₉, C(-CN)OR₉, CR₁₄C(O)OR₈ hat, oder =Z eine 2-(1,3-Dithian)-, Dimethylthioketal-, 2-(1,3-Dioxolan)- oder Dimethylketalgruppe ist;
R₇ ein Rest -(CR₄R₅)_{q}R₁₂ oder ein C₁₋₆-Alkylrest ist, wobei der Rest R₁₂ oder der C₁₋₆-Alkylrest gegebenenfalls ein- oder mehrmals mit einem durch ein bis drei Fluoratome substituierten C₁₋₂-Alkylrest, -F, -Br, -Cl, -NO₂, einen Rest -Si(R₄)₃, -NR₁₀R₁₁, -C(O)R₈, -CO₂R₈, -OR₈, -CN, -C(O)NR₁₀R₁₁, -OC(O)NR₁₀R₁₁, -OC(O)R₈, -NR₁₀C(O)NR₁₀R₁₁, -NR₁₀C(O)R₁₁, -NR₁₀C(O)OR₉, -NR₁₀C(O)R₁₃, -C(NR₁₀)NR₁₀R₁₁, -C(NCN)NR₁₀R₁₁, -C(NCN)SR₉, -NR₁₀C(NCN)SR₉, -NR₁₀C(NCN)NR₁₀R₁₁, -NR₁₀S(O)₂R₉, -S(O)_{m'}R₉, -NR₁₀C(O)C(O)NR₁₀R₁₁, -NR₁₀C(O)C(O)R₁₀, oder eine Thiazolyl-, Imidazolyl-, Oxazolyl-, Pyrazolyl-, Triazolyl- oder Tetrazolyl-Gruppe substituiert ist;
q gleich 0, 1 oder 2 ist;
R₁₂ ein C₃₋₇-Cycloalkylrest, eine (2-, 3- oder 4-Pyridyl-) , (1- oder 2-Imidazolyl-), Piperazinyl-, Morpholinyl-, (2- oder 3-Thienyl-), (4- oder 5-Thiazolyl-) oder Phenylgruppe ist;
R₈ unabhängig aus einem Wasserstoffatom oder R₉ ausgewählt ist;
R₉ ein C₁₋₄-Alkylrest ist, der gegebenenfalls durch ein oder drei Fluoratome substituiert ist;
R₁₀ die Bedeutung OR₈ oder R₁₁ hat;
R₁₁ ein Wasserstoffatom oder ein C₁₋₄-Alkylrest ist, der gegebenenfalls durch ein bis drei Fluoratome substituiert ist; oder wenn R₁₀ und R₁₁ als NR₁₀R₁₁ vorliegen, sie dann gemeinsam mit dem Stickstoff einen 5- bis 7-gliedrigen Ring bilden können, der gegebenenfalls mindestens ein zusätzliches, aus O, N oder S ausgewähltes Heteroatom, enthält;
R₁₃ eine Oxazolidinyl-, Oxazolyl-, Thiazolyl-, Pyrazolyl-, Triazolyl-, Tetrazolyl-, Imidazolyl-, Imidazolidinyl-, Thiazolidinyl-, Isoxazolyl-, Oxadiazolyl- oder Thiadiazolyl-Gruppe ist, und diese heterocyclischen Ringe jeweils über ein Kohlenstoffatom miteinander verbunden sind und jeweils unsubstituiert oder durch ein oder zwei C₁₋₂-Alkylreste substituiert sein können;
R₁₄ ein Wasserstoffatom oder ein Rest R₇ ist; oder wenn R₈ und R₁₄ als NR₈R₁₄ vorliegen, sie dann zusammen mit dem Stickstoff einen 5- bis 7-gliedrigen Ring bilden können, der ein oder mehrere zusätzliche, aus O, N oder S ausgewählte Heteroatome enthält;
R₁₅ die Bedeutung C(O)R₁₄, C(O)NR₄R₁₄, S(O)₂R₇ oder S(O)₂NR₄R₁₄ hat,
vorausgesetzt, dass wenn R₁₂ eine N-Imidazolyl-, N-Triazolyl-, N-Pyrrolyl-, N-Piperazinyl- oder N-Morpholinylgruppe ist, q dann nicht gleich 1 ist;
oder die pharmazeutisch verträglichen Salze davon.

3. Verbindung nach Anspruch 1 oder 2, wobei R₁ eine -CH₂-Cyclopropylgruppe, ein -CH₂-C₅₋₆-Cycloalkyl-, -C₄₋₆-Cycloalkylrest, eine Tetrahydrofuran-3-yl-, (3- oder 4-Cyclopentenyl-), Benzylgruppe oder ein -C₁₋₂-Alkylrest ist, der gegebenenfalls durch 1 oder mehrere Fluoratome substituiert ist, oder (CH₂)₂₋₄-OH ist; R₂ eine Methylgruppe oder ein fluorsubstituierter Alkylrest ist, R₃ die Bedeutung CN oder C≡CR₈ hat, und X ein Rest YR₂ ist.

4. Verbindung nach einem der Ansprüche 1 bis 3, wobei R₁ eine -CH₂-Cyclopropyl-, Cyclopentyl-, Methylgruppe oder ein Rest CF₂H ist; R₃ die Bedeutung CN oder C≡CH hat, X ein Rest YR₂ ist; Y ein Sauerstoffatom ist; X₂ ein Sauerstoffatom ist, X₃ ein Wasserstoffatom ist ; und R₂ eine Gruppe CF₂H oder eine Methylgruppe ist.

5. Verbindung nach Anspruch 1, nämlich 3-Cyano-3-(cyclopentyloxy-4-methoxyphenyl)cyclohexan-1-on.

6. Arzneimittel, umfassend eine Verbindung der Formel (I) nach einem der Ansprüche 1-5 und einen pharmazeutisch verträglichen Exzipienten.

7. Verwendung einer Verbindung nach einem der Ansprüche 1-5 zur Herstellung eines Medikamentes zur Verwendung bei der Behandlung eines allergischen oder entzündlichen Zustands.

8. Verwendung nach Anspruch 7, wobei das hergestellte Medikament zur Behandlung von Asthma verwendet wird.

## Revendications

1. Composé de formule (I) : 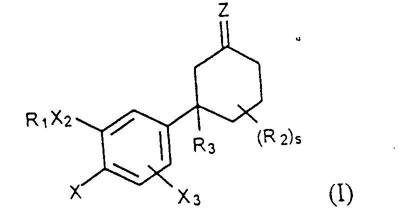 dans laquelle :
R₁ représente un groupe - (CR₄R₅)ₙC(O)O(CR₄R₅)ₘR₆, - (CR₄R₅)ₙC(O)NR₄(CR₄R₅)ₘR₆, -(CR₄R₅)ₙO(CR₄R₅)ₘR₆ ou -(CR₄R₅)ᵣR₆ dans lequel les groupements alkyle peuvent être facultativement substitués avec un ou plusieurs halogènes ;
m a une valeur de 0 à 2 ;
n a une valeur de 1 à 4 ;
r a une valeur de 0 à 6 ;
R₄ et R₅ sont choisis, indépendamment, entre l'hydrogène et un groupe alkyle en C₁ ou C₂ ;
R₆ représente l'hydrogène, un groupe méthyle, hydroxyle, aryle, aryle à substituant halogéno, aryloxy(alkyle en C₁ à C₃), aryloxy(alkyle en C₁ à C₃) à substituant halogéno, indanyle, indényle, polycycloalkyle en C₇ à C₁₁, tétrahydrofurannyle, furannyle, tétrahydropyrannyle, pyrannyle, tétrahydrothiényle, thiényle, tétrahydrothiopyrannyle, thiopyrannyle, cycloalkyle en C₃ à C₆ ou un groupe cycloalkyle en C₄ à C₆ contenant une ou deux liaisons insaturées, dans lequel les groupements cycloalkyle hétérocycliques peuvent être facultativement substitués avec 1 à 3 groupes méthyle ou un groupe éthyle ;
sous réserve que :
a) lorsque R₆ représente un groupe hydroxyle, alors m soit égal à 2 ; ou
b) lorsque R₆ représente un groupe hydroxyle, alors r ait une valeur de 2 à 6 ; ou
c) lorsque R₆ représente un groupe 2-tétrahydropyrannyle, 2-tétrahydrothiopyrannyle, 2-tétrahydrofurannyle ou 2-tétrahydrothiényle, alors m soit égal à 1 ou 2 ; ou
d) lorsque R₆ représente un groupe 2-tétrahydropyrannyle, 2-tétrahydrothiopyrannyle, 2-tétrahydrofurannyle ou 2-tétrahydrothiényle, alors r ait une valeur de 1 à 6 ;
e) lorsque n est égal à 1 et m est égal à 0, alors R₆ soit autre que H dans le groupe -(CR₄R₅)ₙO(CR₄R₅)ₘR₆ ;
X représente un groupe YR₂, halogéno, nitro, NR₄R₅ ou formylamine ;
Y représente O ou un groupe S(O)ₘ' ;
m' est égal à 0, 1 ou 2 ;
X₂ représente O ou un groupe NR₈ ;
X₃ représente l'hydrogène ou X ;
R₂ est choisi, indépendamment, entre un groupe -CH₃ et un groupe -CH₂CH₃ facultativement substitués avec un ou plusieurs halogènes ;
s a une valeur de 0 à 4 ;
R₃ représente un groupe alkyle en C₁ à C₄, alkyle en C₁ à C₄ substitué avec le fluor, CH₂NHC (O) C (O) NH₂, -CH=CR_{8'}R_{8'}, cyclopropyle facultativement substitué avec un substituant R_{8'}, CN, CH₂OR₈, CH₂NR₈R₁₀, C(Z')H, C(O)OR₈, C(O)NR₈R₁₀ ou C≡CR₈, ;
Z' représente 0, un groupe NR₉, NOR₈, NCN, C(-CN)₂, CR₈CN, CR₈NO₂, CR₈C(O)OR₈, CR₈C(O) NR₈R₈, C(-CN)NO₂, C(-CN)C(O)OR₉ ou C(-CN)C(O)NR₈R₈ ;
Z représente 0, un groupe NR₇, NCR₄R₅ (alcényle en C₂ à C₆), NOR₁₄, NOR₁₅, NOCR₄R₅ (alcényle en C₂ à C₆), NNR₄R₁₄, NNR₄R₁₅, NCN, NNR₈C(O)NR₈R₁₄, NNR₈C (S) NR₈R₁₄, C(-CN)₂, CR₁₄CN, CR₁₄C(O)OR₈, CR₁₄C(O)NR₈R₁₄, C(-CN)NO₂, C(-CN)C(O)OR₉, C(-CN)OC(O)R₉, C(-CN)OR₉, C(-CN)C(O)NR₈R₁₄ ou bien =Z représente un groupe 2-(1,3-dithiane), 2-(1,3-dithiolane), diméthylthiocétal, diéthylthiocétal, 2-(1,3-dioxolanne), 2-(1,3-dioxanne), 2-(1,3-oxathiolane), diméthylcétal ou diéthylcétal ;
R₇ représente un groupe - (CR₄R₅)_{q}R₁₂ ou alkyle en C₁ à C₆ dans lequel le groupe R₁₂ ou le groupe alkyle en C₁ à C₆ est facultativement substitué une ou plusieurs fois avec un substituant alkyle en C₁ ou C₂ facultativement substitué avec un à trois atomes de fluor, -F, -Br, -Cl, -NO₂, -Si(R₄)₃, -NR₁₀R₁₁, -C(O)R₈, -CO₂R₈, -OR₈, -CN, -C(O)NR₁₀R₁₁, -OC(O)NR₁₀R₁₁, -OC(O)R₈, -NR₁₀C(O)NR₁₀R₁₁, -NR₁₀C(O)R₁₁, -NR₁₀C(O)OR₉, -NR₁₀C(O)R₁₃, -C(NR₁₀)NR₁₀R₁₁, -C(NCN)NR₁₀R₁₁, -C(NCN)SR₉, -NR₁₀C(NCN)SR₉, -NR₁₀C(NCN)NR₁₀R₁₁, -NR₁₀S(O)₂R₉, -S(O)_{m'}R₉, -NR₁₀C(O)C(O)NR₁₀R₁₁, -NR₁₀C(O)C(O)R₁₀, thiazolyle, imidazolyle, oxazolyle, pyrazolyle, triazolyle ou tétrazolyle ;
q est égal à 0, 1 ou 2 ;
R₁₂ représente un groupe cycloalkyle en C₃ à C₇, (2-,3-ou 4-pyridyle), pyrimidyle, pyrazolyle, (1- ou 2-imidazolyle), thiazolyle, triazolyle, pyrrolyle, pipérazinyle, pipéridinyle, morpholinyle, furannyle, (2- ou 3-thiényle), (4- ou 5-thiazolyle), quinolinyle, naphtyle ou phényle ;
R₈ est choisi indépendamment entre l'hydrogène et un groupe R₉ ;
R_{8'} représente un groupe R₈ ou le fluor ;
R₉ représente un groupe alkyle en C₁ à C₄ facultativement substitué avec un à trois atomes de fluor ;
R₁₀ représente un groupe OR₈ ou R₁₁ ;
R₁₁ représente l'hydrogène, ou un groupe alkyle en C₁ à C₄ facultativement substitué avec un à trois atomes de fluor, ou bien, lorsque R₁₀ et R₁₁ sont sous forme d'un groupe NR₁₀R₁₁, ils peuvent, conjointement avec l'atome d'azote, former un noyau penta- à heptagonal contenant facultativement au moins un hétéroatome supplémentaire choisi entre O, N et S ;
R₁₃ représente un groupe oxazolidinyle, oxazolyle, thiazolyle, pyrazolyle, triazolyle, tétrazolyle, imidazolyle, imidazolidinyle, thiazolidinyle, isoxazolyle, oxadiazolyle ou thiadiazolyle, chacun de ces noyaux hétérocycliques étant connecté par un atome de carbone et chacun pouvant être non substitué ou substitué avec un ou deux groupes alkyle en C₁ ou C₂ ;
R₁₄ représente l'hydrogène ou un groupe R₇ ; ou bien, lorsque R₈ et R₁₄ sont sous la forme NR₈R₁₄, ils peuvent, conjointement avec l'atome d'azote, former un noyau penta- à heptagonal contenant facultativement un ou plusieurs hétéroatomes supplémentaires choisis entre O, N et S ;
R₁₅ représente un groupe C(O)R₁₄, C(O)NR₄R₁₄, S(O)₂R₇ ou S(O)₂NR₄R₁₄ ;
sous réserve que :
(f) lorsque Z représente O, X₂ représente l'oxygène, X₃ représente l'hydrogène, s est égal à 0 et X représente un groupe YR₂, alors R₃ soit autre que l'hydrogène ;
(g) lorsque R₁₂ représente un groupe N-pyrazolyle, N-imidazolyle, N-triazolyle, N-pyrrolyle, N-pipérazinyle, N-pipéridinyle ou N-morpholinyle, alors q ne soit pas égal à 1 ; ou
(h) lorsque Z représente O ou =Z représente un groupe 2-(1,3-dioxolanne) et R₃ représente un groupe CH₃, CH₂OH ou CH₂O(alkyle en C₁ à C₄), alors R₁X₂ ne représente pas un groupe alkoxy en C₁ à C₃ et X ne représente pas un groupe halogéno, méthoxy, éthoxy, méthylthio ou éthylthio ; ne consistant pas en 3-cyano-3-(3,4-diméthoxyphényl)cyclohexane-1-one ;
ou ses sels pharmaceutiquement acceptables.

2. Composé suivant la revendication 1, de formule (Ia) 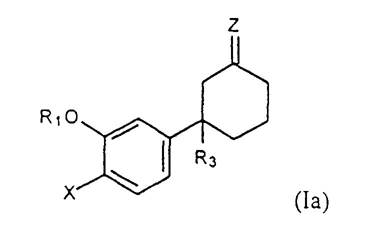 dans laquelle :
R₁ représente un groupe CH₂-cyclopropyle, CH₂(cycloalkyle en C₅ ou C₆), cycloalkyle en C₄ à C₆, polycycloalkyle en C₇ à C₁₁, (3- ou 4-cyclopentényle), phényle, tétrahydrofuranne-3-yle, benzyle ou alkyle en C₁ ou C₂ facultativement substitué avec un ou plusieurs atomes de fluor, -(CH₂)₁₋₃C(O)O(CH₂)₀₋₂CH₃, - (CH₂)₁₋₃O (CH₂)₀₋₂CH₃ ou -(CH₂)₂₋₄OH ;
X représente un groupe YR₂, halogéno, nitro, NR₄R₅ ou formylamine ;
Y représente O ou un groupe S(O)_{m'} ;
m' est égal à 0, 1 ou 2 ;
R₂ représente un groupe -CH₃ ou -CH₂CH₃ facultativement substitué avec un ou plusieurs halogènes ;
R₃ représente un groupe alkyle en C₁ à C₄, alkyle en C₁ à C₄ à substituant halogéno, CH₂NHC(O)C(O)NH₂, CN, CH₂OR₈, C(Z')H, C(O)OR₈, C(O)NR₈R₁₀ ou C≡CR_{8'} ;
Z' représente O ou un groupe NOR₈ ;
Z représente O, un groupe NR₇, NOR₁₄, NOR₁₅, NNR₄R₁₄, NNR₄R₁₅, NCN, C(CN)₂, C(-CN)OC(O)R₉, C-CN)OR₉, CR₁₄C(O)OR₈, ou bien =Z représente un groupe 2-(1,3-dithiane), diméthylthiocétal, 2-(1,3-dioxolanne) ou diméthylcétal ;
R₇ représente un groupe -(CR₄R₅)_{q}R₁₂ ou alkyle en C₁ à C₆ dans lequel le groupe R₁₂ ou alkyle en C₁ à C₆ est facultativement substitué une ou plusieurs fois avec un substituant alkyle en C₁ ou C₂ facultativement substitué avec un à trois atomes de fluor, -F, -Br, -Cl, -NO₂, -Si(R₄)₃, -NR₁₀R₁₁, -C(O)R₈, -CO₂R₈, -OR₈, -CN, -C(O)NR₁₀R₁₁, -OC(O)NR₁₀R₁₁, -OC(O)R₈, -NR₁₀C(O)NR₁₀R₁₁, -NR₁₀C(O)R₁₁, -NR₁₀C(O)OR₉, -NR₁₀C(O)R₁₃, -C (NR₁₀)NR₁₀R₁₁, -C (NCN) NR₁₀R₁₁, -C(NCN)SR₉, -NR₁₀C(NCN)SR₉, -NR₁₀C (NCN) NR₁₀R₁₁, -NR₁₀S(O)₂R₉, -S(O)_{m'} R₉, -NR₁₀C(O)C(O)NR₁₀R₁₁, -NR₁₀C(O)C(O)R₁₀, thiazolyle, imidazolyle, oxazolyle, pyrazolyle, triazolyle ou tétrazolyle ;
q est égal à 0, 1 ou 2 ;
R₁₂ représente un groupe cycloalkyle en C₃ à C₇, (2-, 3- ou 4-pyridyle), (1- ou 2-imidazolyle), pipérazinyle, morpholinyle, (2- ou 3-thiényle), (4- ou 5-thiazolyle), ou phényle ;
R₈ est choisi indépendamment entre l'hydrogène et un groupe R₉ ;
R₉ représente un groupe alkyle en C₁ à C₄ facultativement substitué avec un à trois atomes de fluor ;
R₁₀ représente un groupe OR₈ ou R₁₁ ;
R₁₁ représente l'hydrogène, ou un groupe alkyle en C₁ à C₄ facultativement substitué avec un à trois atomes de fluor ; ou bien, lorsque R₁₀ et R₁₁ sont sous la forme NR₁₀R₁₁, ils peuvent, conjointement avec l'atome d'azote, former un noyau penta- à heptagonal contenant facultativement au moins un hétéroatome supplémentaire choisi entre O, N et S ;
R₁₃ représente un groupe oxazolidinyle, oxazolyle, thiazolyle, pyrazolyle, triazolyle, tétrazolyle, imidazolyle, imidazolidinyle, thiazolidinyle, isoxazolyle, oxadiazolyle ou thiadiazolyle, chacun de ces noyaux hétérocycliques étant connecté par un atome de carbone et chacun pouvant être non substitué ou substitué avec un ou deux groupes alkyle en C₁ ou C₂ ;
R₁₄ représente l'hydrogène ou un groupe R₇ ; ou bien, lorsque R₈ et R₁₄ sont sous la forme NR₈R₁₄, ils peuvent, conjointement avec l'atome d'azote, former un noyau penta- à heptagonal contenant facultativement un ou plusieurs hétéroatomes supplémentaires choisis entre O, N et S ;
R₁₅ représente un groupe C(O)R₁₄, C(O)NR₄R₁₄, S(O)₂R₇ ou S(O)₂NR₄R₁₄ ;
sous réserve que, lorsque R₁₂ représente un groupe N-imidazolyle, N-triazolyle, N-pyrrolyle, N-pipérazinyle ou N-morpholinyle, alors q ne soit pas égal à 1 ;
ou ses sels pharmaceutiquement acceptables.

3. Composé suivant la revendication 1 ou 2, dans lequel R₁ représente un groupe -CH₂-cyclopropyle, -CH₂(cycloalkyle en C₅ ou C₆), -cycloalkyle en C₄ à C₆, tétrahydrofuranne-3-yle, (3- ou 4-cyclopentényle), benzyle ou -alkyle en C₁ ou C₂ facultativement substitué avec un ou plusieurs atomes de fluor, ou bien -(CH₂)₂₋₄OH ; R₂ représente un groupe méthyle ou alkyle à substituant fluoro, R₃ représente un groupe CN ou C≡CR₈, et X représente un groupe YR₂.

4. Composé suivant l'une quelconque des revendications 1 à 3, dans lequel R₁ représente un groupe -CH₂-cyclopropyle, cyclopentyle, méthyle ou CF₂H ; R₃ représente un groupe CN ou C≡CH ; X représente un groupe YR₂ ; Y représente l'oxygène ; X₂ représente l'oxygène ; X₃ représente l'hydrogène ; et R₂ représente un groupe CF₂H ou méthyle.

5. Composé suivant la revendication 1, qui est la 3-cyano-3-(cyclopentyloxy-4-méthoxyphényl)cyclohexane-1-one.

6. Composition pharmaceutique comprenant un composé de formule (I) suivant l'une quelconque des revendications 1 à 5 et un excipient pharmaceutiquement acceptable.

7. Utilisation d'un composé suivant l'une quelconque des revendications 1 à 5, pour la production d'un médicament destiné à être utilisé dans le traitement d'un état allergique ou inflammatoire.

8. Utilisation de la manière décrite dans la revendication 7, dans laquelle le médicament qui a été produit est utilisé pour le traitement de l'asthme.
